# EUROPÄISCHE PATENTSCHRIFT

(11) **EP 0 327 797 B1**
(45) Veröffentlichungstag und Bekanntmachung des Hinweises auf die Patenterteilung: **03.09.2003**
(21) Anmeldenummer: 89100104.2
(22) Anmeldetag: 04.01.1989
(51) Int. Cl.: C12P 21/00, C12N 15/00

(54) **Verfahren zur Herstellung von Proteinen oder proteinhaltigen Genprodukten**
Method for the preparation of proteins or protein-containing gene products
Méthode pour la préparation de protéines ou de produits de gène contenant des protéines

(30) Priorität: 05.01.1988 DE 3800134; 17.02.1988 DE 3804890
(43) Veröffentlichungstag der Anmeldung: 16.08.1989
(73) Patentinhaber: Roche Diagnostics GmbH, 68298 Mannheim (DE)
(72) Erfinder: Wolf, Dieter Heinrich, Prof. Dr., D-7803 Gundelfingen (DE); Kopetzki, Erhard, Dr. rer. nat., D-8122 Penzberg (DE); Schumacher, Günther, Dr. rer. nat., D-8139 Bernried (DE)

(56) Entgegenhaltungen:
- WO-A-85/03949
- YEAST, Band 1, 1985, Seiten 139-157, John Wiley & Sons Ltd, Chicester, GB; T. ACHSTETTER et al.: "Proteinases, proteolysis and biological control in the yeast Saccharomyces cerevisiae"
- THE EMBO JOURNAL, Band 6, Nr. 7, 1987, Seiten 2157-2163, IRL Press LTD, Oxford, GB; B. MECHLER et al.: "Maturation of vacuolar (lysosomal) enzymes in yeast: proteinase yscA and proteinase yscB are catalysts of the processing and activation event of carboxypeptidase yscY"

## Beschreibung

Die Erfindung betrifft ein Verfahren zur Herstellung von Proteinen oder proteinhaltigen Genprodukten auf gentechnologischem Wege durch Transformation von eukaryontischen Wirtszellen mit einem das Gen für das gewünschte Protein enthaltenden rekombinanten DNA-Molekül, Kultivierung der Zellen und Isolierung des Genprodukts nach an sich bekannten Methoden.

Die Bestimmung klinisch-chemischer Parameter erfolgt heutzutage in großem Umfang mit enzymatischen Methoden. Die für die Herstellung der dafür benötigten Reagenzien verwendeten Enzyme werden aus verschiedenen Quellen pflanzlichen oder tierischen Ursprungs oder aus Mikroorganismen gewonnen.

Zur Herstellung von Enzymen und anderen Proteinen gewinnen Mikroorganismen hierbei zunehmend an Bedeutung, da nur diese in praktisch beliebiger Menge durch Fermentation verfügbar gemacht werden können und somit die Isolierung größerer Proteinmengen ermöglichen. Besondere Bedeutung besitzen hierbei bekanntermaßen Hefen, wie z.B. Saccharomyces cerevisiae, da in diesen Organismen die homologe Expression von Proteinen ebenso möglich ist wie die heterologe Expression eukaryontischer, z.B. therapeutisch wichtiger Proteine. In E. coli dagegen, dem am häufigsten benutzten Wirtsorganismus, unterscheiden sich viele heterolog exprimierte Proteine von ihren in einem homologen System exprimierten natürlichen Gegenstücken und sind biologisch nicht aktiv, oder fallen als unlösliche inaktive Proteinaggregate, "refractile bodies" genannt, an. Viele eukaryontische Proteine, die in E.coli inaktiv exprimiert werden, werden in S. cerevisiae löslich und aktiv gebildet (Biotechnology and Genetic Engineering Reviews 3 (1985) 377-416). Dies mag unter anderem dadurch bedingt sein, daß Hefen über die typischen eukaryontischen posttranslationalen Modifizierungssysteme, wie z.B. "Proteinfaltung", Proteinreifung, Glycosylierung und Acetylierung verfügen, zur Sekretion fähig sind und die Ausbildung von Disulfidbrücken in Polypeptiden und Proteinen ermöglichen. Außerdem sind Hefen nicht pathogen und im Gegensatz zu E.coli frei von Toxinen und pyrogenen Zellwandbestandteilen.

Die Hefe ist einer der ältesten Kulturorganismen des Menschen. Sie wurde und wird noch immer hauptsächlich zur alkoholischen Gärung (Wein, Bier usw.) und als "Backhilfe" bei der Zubereitung von Teigwaren verwendet. Daneben hat die Hefe industrielle Bedeutung als kostengünstige Rohstoffquelle zur Isolierung von niedermolekularen Substanzen wie z.B. NAD, ATP und Glutathion, und hochmolekularen Substanzen wie z.B. DNA, RNA und vor allem Enzymen, wie z.B. Alkohol-Dehydrogenase, Aldehyd-Dehydrogenase, Acetyl-CoA-Synthetase, α-Glucosidase, Glycerinaldehyd-3-phosphatdehydrogenase, Glucose-6-phosphat-dehydrogenase und Hexokinase, erlangt. Hefe ist leicht zu kultivieren und aufgrund von langjährigen Erfahrungen im industriellen Maßstab einfach zu fermentieren. Die Hefe, ein zu den niederen Eukaryonten zählender Einzeller, besitzt die typischen Merkmale eines Eukaryonten, ist aber trotzdem genetischen Untersuchungen und genetischen Manipulationen leicht zugänglich, wodurch sie besonders als Wirtsorganismus im Hinblick auf rekombinante DNA-Technologie geeignet ist, d.h. zur homologen und heterologen Expression von biologisch aktiven Polypeptiden und Proteinen.

Bei der Expression von Proteinen in Hefe ist in vielen Fällen jedoch die Menge an gebildetem Protein nicht befriedigend. Häufig findet man, daß nach Erreichen der frühstationären Wachstumsphase die spezifische Aktivität eines gewünschten Proteins im Verlauf der stationären Wachstumsphase wieder abnimmt, obwohl die gebildete Biomasse der Mikroorganismen noch ansteigt. Dies ist u.a. auf einen proteolytischen Angriff wirtsspezifischer Proteasen auf die gebildeten Proteine zurückzuführen.

Aufgabe der vorliegenden Erfindung war es daher, ein Verfahren zur Herstellung von Proteinen auf gentechnologischem Wege bereitzustellen, mit dem auch in der stationären Phase des Wachstums Proteine gebildet und stabil akkumuliert werden können und damit die Ausbeute des Fermentationsverfahrens gesteigert werden kann.

Diese Aufgabe wird erfindungsgemäß gelöst durch ein Verfahren zur Herstellung von Proteinen oder proteinhaltigen Genprodukten durch Transformation von eukaryontischen Wirtszellen mit einem das Gen für das gewünschte Protein enthaltenden rekombinanten DNA-Molekül, Kultivierung der Zellen und Isolierung des Genproduktes nach der Expression, welches dadurch gekennzeichnet ist, daß man als Wirtszellen einen Hefestamm verwendet, der in der stationären Züchtungsphase defizient an den Proteasen A und B ist.

Bevorzugt ist der Hefestamm zusätzlich defizient an der Protease D.

In einer weiteren bevorzugten Ausführungsform wird ein Hefestamm verwendet, der zusätzlich zu den Defizienzen an den Proteasen A, B und gegebenenfalls D, defizient an zumindest einer der Carboxypeptidasen Y und S ist. Die Bezeichnung der Proteasen und Carboxypeptidasen in dieser Anmeldung entspricht ihrer Bezeichnung in Yeast 1 (1985) 139-154.

Durch die Verwendung dieser Protease-defizienten Hefestämme im erfindungsgemäßen Verfahren wird es ermöglicht, Proteine oder proteinhaltige Genprodukte in Hefe in erhöhten Ausbeuten herzustellen, ohne daß auch bei extrem langen Fermentationszeiten oder bei der anschließenden Isolierung nach an sich bekannten Methoden ein proteolytischer Angriff, und damit eine Inaktivierung der Proteine, stattfindet.

In einer besonders bevorzugten Ausführungsform der Erfindung verwendet man als Wirtszellen den Hefestamm ABYSD-11, DSM 4322. Dieser Wirtsstamm ist bezüglich der Proteasen A, B, D und der Carboxypeptidasen Y und S defizient. Zusätzlich weist er Auxotrophien in der Adenin-, Histidin- und Lysin-Biosynthese auf. Auxotrophie bedeutet die Unfähigkeit von Mikroorganismen (meist Mutanten von Bakterien oder Hefen), bestimmte Wachstumsfaktoren, wie z.B. Aminosäuren, aus einfachen Vorläufern synthetisieren zu können. Im Gegensatz zu den entsprechenden Wildtypstämmen wachsen auxotrophe Mutanten dabei nicht auf sogenannten Minimalmedien. Stattdessen benötigen sie ein Vollmedium, das die für das Wachstum notwendigen Komponenten, die sie nicht selbst synthetisieren können, enthält. Mikroorganismen können für einen, aber auch mehrere Wachstumsfaktoren auxotroph sein (E.-L. Winnacker, Gene und Klone, 1985, Verlag Chemie, Appendix C).

In einer weiteren bevorzugten Ausgestaltung der Erfindung kreuzt man den Protease-defizienten Hefestamm mit einem anderen, auxotrophen oder/und Chemikalien-sensitiven Hefestamm, isoliert nach Sporulation durch Selektionierung über die Auxotrophie oder/und Sensitivität aus den entstandenen Hybridstämmen einen Hefestamm, der mindestens defizient an den Protease A und B ist und mindestens eine der Auxotrophien oder/und Chemikaliensensitivitäten der Elternstämme aufweist und verwendet diesen Hybridstamm als Wirtszellen.

Unter Chemikaliensensitivität versteht man die Unfähigkeit eines Microorganismus, in einem Medium zu wachsen, das bestimmte Chemikalien enthält, z.B. Methotrexat, Chloramphenicol und das Gentamycinderivat G418. Erst nach Transformation des Microorganismus mit einer rekombinanten DNA, die ein Gen enthält, das dem Microorganismus Resistenz gegen diese Chemikalien verleiht (Dehydrofolatreductase, DHFR; Chloramphenicol-Acetyltransferase, CAT; Transposon Tn601 kodierte Aminoglykosid-Phosphotransferase etc.) kann der Microorganismus in dem Medium wachsen. Die Kreuzung und Sporulation kann beispielsweise analog Sherman et al., Methods in Yeast Genetics, Cold Spring Harbor Laboratory, Cold Spring Harbor, New York, 1984, durchgeführt werden. In einer besonders bevorzugten Ausführungsform der Erfindung kreuzt man hierzu den Hefestamm ABYSD-11, DSM 4322, (a pra1, prb1, prc1, prd1, cps1, ade lys his7), mit entweder dem Hefestamm TCY2824-1A (α mal1S-Δ ura3-52 his4), DSM 4317, oder DBY 746, DSM 4316, (α his3-Δ1 leu2-3 leu2-112 ura3-52 trp1-289a), welche ein defektes Maltasestrukturgen und Auxotrophien im Uracil- und Histidin-, bzw. Auxotrophien im Histidin-, Leucin-, Uracil- und Tryptophan-Biosyntheseweg aufweisen. Bevorzugt isoliert man sodann Hybridstämme, die defizient an den Proteasen A, B und gegebenenfalls D, und an den Carboxypeptidasen Y und S sind und zusätzlich mindestens eine bzw. mehrere Auxotrophien wie z.B. die Uracil-, Lysin- und Maltaseverwertungsauxotrophien, oder die Leucin- und Tryptophan-, oder schließlich die Leucin-, Uracil- und Histidin-Auxotrophien der Elternstämme aufweisen.

In einer weiteren bevorzugten Ausführungsform der Erfindung enthält das rekombinante DNA-Molekül daher zusätzlich zu dem Gen für das gewünschte Protein oder proteinhaltige Genprodukt ein oder mehrere Gene, die die Auxotrophien oder/und Chemikaliensensitivitäten des Wirtsstammes komplementieren.

Durch die bevorzugte Ausführungsform der Erfindung wird eine einfache Unterscheidung von transformierten und nicht transformierten Wirtszellen ermöglicht. Die Anwesenheit und Expression von Genen, die eine oder mehrere der Auxotrophien oder/und Chemikaliensensitivitäten des Wirtsstammes komplementieren, ermöglicht nämlich transformierten Zellen, auch auf Medien zu wachsen, die beispielsweise eine Aminosäure nicht enthalten, die der Wirtsstamm selbst nicht synthetisieren kann, deren Gen jedoch auf dem rekombinanten DNA-Molekül vorhanden ist. Nicht-transformierte Wirtsstämme, welche also das rekombinante DNA-Molekül und das darauf enthaltene Auxotrophie-komplementierende Gen nicht aufweisen, können dagegen in einem derartigen Medium nicht wachsen. Hierdurch kann auf einfache Weise eine Selektionierung auf transformierte Wirtszellen vorgenommen werden, wodurch auch die Gefahr eines Verlustes der rekombinanten DNA, welche das Gen für das gewünschte Protein enthält, während der Fermentation vermieden wird, da kein Wachstumsvorteil von nichttransformierten Zellen besteht.

Alternativ ist es auch möglich, die Auxotrophie oder/und Chemikaliensensitivität eines Wirtsstammes durch Einbringen eines weiteren zusätzlichen rekombinanten DNA-Moleküls, das ein oder mehrere Gene enthält, die die Auxotrophien bzw. Chemikaliensensitivitäten der Wirtszellen komplementieren, zu überwinden. Dabei kann jedoch die einfache Möglichkeit der Selektionierung auf das Gen für das gewünschte Produkt enthaltende Wirtszellen nicht wahrgenommen werden.

Für das rekombinante DNA-Molekül kommen alle rekombinanten DNA-Moleküle in Frage, mit denen Hefezellen transformiert werden können, und die zur Expression eines Fremdgens fähig sind. Hierbei kommt nicht nur die extrachromosomale Transkription beispielsweise eines Plasmids in Frage, sondern es ist ebenfalls möglich, das Gen für das gewünschte Protein über einen Integrationsvektor oder ein integrierendes DNA-Fragment, die jeweils eine vollständige Expressionskassette (Promotor, Terminator, Regulator, Transkriptionsverstärker u.ä.) enthalten, in das Hefegenom einzuschleusen und zusammen mit den hefeeigenen Proteinen zu exprimieren. Voraussetzung hierfür ist das Vorhandensein von Homologien auf dem rekombinanten DNA-Molekül und chromosomalen Hefesequenzen. Über solche homologe Regionen kann ein integrierendes DNA-Fragment nach an sich bekannten Methoden in das Hefechromosom eingeschleust werden.

In einer weiteren bevorzugten Ausführungsform der Erfindung ist daher das rekombinante DNA-Molekül entweder ein Plasmid oder aber ein Integrationsvektor oder ein integrierendes DNA-Fragment. Als Plasmide sind hierbei wiederum besonders bevorzugt Hefeplasmide, die in hoher Kopienzahl in der Zelle vorkommen. Solche Hefeplasmide sind beispielsweise hybride Hefe/E. coli-Vektoren ("Shuttle-Vektoren"), die als YRp, YEp, YIp und YCp bezeichnet werden. In hoher Kopienzahl kommen in der Zelle wiederum nur die YEp- und YRp-Plasmide vor. Diese sind nämlich aufgrund des Vorhandenseins von Sequenzen, die eine eigenständige Replikation der Plasmide ermöglichen, unabhängig von der Replikation des Hefechromosoms und liegen normalerweise in Stückzahlen von 5 bis 40 Kopien pro Zelle vor. Die YIp-Plasmide können nur durch Integration in das Hefegenom zur Expression gebracht werden. Sie sind daher beispielhaft. für Integrationsvektoren. YIp-Plasmide weisen eine zehnmal niedrigere Transformationsrate, dafür aber eine bedeutend größere Stabilität im Vergleich zu YRp- und YEp-Plasmiden auf. YRp- und YEp-Plasmide können ohne Selektionsdruck bei der Zellvermehrung verloren werden (Nature 305 (1983) 391-397). Ein solcher Selektionsdruck besteht aber bei der bevorzugten Ausführungsform der Erfindung, bei der die Fermentation von auxotrophen oder Chemikalien-sensitiven Wirtsstämmen in Minimalmedien, oder Medien, die eine bestimmte Chemikalie enthalten, für die der Wirtsstamm sensitiv ist, durchgeführt wird und die rekombinante DNA zusätzlich ein oder mehrere Gene aufweist, die'die Auxotrophie oder Sensitivität komplementieren.

Die vorliegende Erfindung ermöglicht die Expression von homologen oder heterologen Proteinen oder proteinhaltigen Genprodukten in hoher Ausbeute und proteolytisch nicht angegriffener Form, wobei auch die Selektionierung auf transformierte Zellen, und dadurch ebenfalls eine Erhöhung der Ausbeute des gewünschten Genproduktes, in einfacher Weise durchgeführt werden kann. Auch beim Aufschließen der Zellmasse und der weiteren Prozedur zur Gewinnung des Genprodukts nach an sich bekannten Methoden erfolgt aufgrund der Proteasedefizienz der Wirtszellen kein proteolytischer Angriff auf das gebildete Produkt.

In einer bevorzugten Ausführungsform der Erfindung wird das Protein alpha-Glucosidase PI hergestellt (siehe auch Beispiel 4).

Die folgenden Beispiele erläutern die Erfindung weiter.

### Beispiel 1

Zur Herstellung der Saccharomycesstämme ABYSMAL81 und ABYSDMAL81 wurde der haploide Saccharomyces cerevisiae-Stamm ABYSD-11 (a pra1 prb1 prc1 prd1, cps1, ade lys his7), DSM 4322, der bzgl. der Proteasen A, B und D und der Carboxypeptidasen Y und S defizient ist, und zusätzlich eine Auxotrophie in der Adenin-, Histidin- und Lysin-Biosynthese besitzt, mit dem Saccharomyces carlsbergensis-Stamm, TCY2824-1A, (α mal1S-Δ ura3-52 his4), DSM 4317, gekreuzt, der durch ein defektes α-Glucosidase-Strukturgen und durch eine Auxotrophie in der Uracil- und Histidin-Biosynthese charakterisiert ist.

Anschließend wurde eine Sporulation durchgeführt und die entstandenen Hefe-Segreganten auf ihre Auxotrophien durch Ausplattieren auf verschiedenen Selektionsmedien und auf ihre Protease-Defizienzen durch Bestimmung der Protease-Aktivitäten in Zellysaten überprüft. Die Stämme ABYSDMAL81 (ura3-52 mal1S-Δ lys pra1 prb1 prc1 prd1 cps1) und ABYSMAL81 (ura3-52 mal1S-Δ lys pra1 prb1 prc1 cps1) wurden identifiziert durch:

### Nachweis der Protease-Defizienzen

Die Segreganten wurden in 5 ml YEPD-Medium (1% Hefeextrakt, 2% Pepton, 2% Glucose) angezogen, die Zellen in der spät-logarithmischen bis früh-stationären Wachstumsphase geerntet, zweimal mit Wasser gewaschen und mit Glasperlen durch Homogenisieren auf einem Whirlmix aufgeschlossen (MGG 145 (1976) 327-333). Die Zellen wurden mit 1 ml 20 mmol/l Tris (HCl), pH 7,0 extrahiert und der Überstand nach Zentrifugation als Rohextrakt weiterverarbeitet. Zur Aktivierung der Proteasen wurde der Rohextrakt auf pH 5,0 titriert und 24 Stunden bei 25°C inkubiert.

### Nachweis der Protease A-Defizienz (pra1)

Keine Hydrolyse durch Zellextrakte von 1,2% säuredenaturiertem Hämoglobin, pH 3,0 (Eur. J. Biochem. 42 (1974) 621-626).

0,5 ml 0,1 mol/l Lactat-Puffer, pH 3,0 mit 1,2% säuredenaturiertem Hämoglobin wurden mit 0,1 ml Zellysat bei 25°C inkubiert. Nach 30 Minuten wurde die Reaktion mit 0,5 ml 10%iger Trichloressigsäure gestoppt und nach Zentrifugation die Trichloressigsäure löslichen Produkte entweder durch Absorptionsmessung bei 280 nm oder durch eine modifizierte Folin-Bestimmung nach McDonald und Chen (Anal. Biochem. 10 (1965) 175-177) bestimmt.

Zur Berechnung der spezifischen Aktivität wurde eine Proteinbestimmung nach Zamenhof durchgeführt (Methods Enzymol. 3 (1957) 702).

Eine Protease A-Defizienz liegt vor, wenn die spezifische hydrolytische Aktivität von Zellysaten gegenüber säuredenaturiertem Hämoglobin auf kleiner 5% im Vergleich mit einem Wildtypstamm herabgesetzt ist.

### Nachweis der Protease B-Defizienz (prb1)

Keine Hydrolyse durch Zellextrakte von 2,4% Azocoll bei pH 7 (Eur. J. Biochem. 42 (1974) 621-626).

0,5 ml einer 2,4%igen Azocoll Suspension in 0,1 mol/l Phosphatpuffer, pH 7,0 wurden mit 0,1 ml Zellysat unter Schütteln bei 25°C inkubiert. Zur Aktivierung der Protease B wurde der Rohextrakt vor der Aktivitätsbestimmung mit Natriumdodecylsulfat (Endkonzentration 0,25%) versetzt.

Nach 30 Minuten wurde die Reaktion durch Zugabe von 0,5 ml 10%iger Trichloressigsäure gestoppt und nach Zentrifugation die Extinktion im Überstand bei 550 nm bestimmt.

Eine Protease B-Defizienz liegt vor, wenn die spezifische hydrolytische Aktivität von Zellysaten gegenüber Azocoll auf kleiner 5% im Vergleich mit einem Wildtypstamm herabgesetzt ist.

### Nachweis der Protease D-Defizienz (prd1)

Keine Hydrolyse durch Zellextrakte von 0,5 mmol/l Bz-Pro-Phe-Arg-NA (Benzoyl-L-prolyl-L-phenylalanyl-L-arginyl-p-nitroanilid) in 50 mmol/l Tris-Maleat-Puffer, pH 7,0 in Gegenwart von Aminopeptidase M (J. Biol. Chem. 260 (1985) 4585-4590)

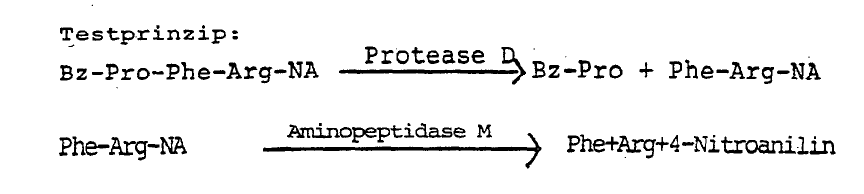

0,03 ml 0,5 mol/l Tris-Maleat Puffer, pH 7,0 wurden mit 0,015 ml 10 mmol/l Bz-Pro-Phe-Arg-NA (gelöst in Dimethylsulfoxid), 10 µl (40 µg, 240 mU) Aminopeptidase M, 0,145 ml Wasser und 0,1 ml Rohextrakt gemischt und die Extinktionsänderung bei 405 nm gegen einen Reagenzienleerwert bestimmt.

Eine Protease D-Defizienz liegt vor, wenn die spezifische hydrolytische Aktivität gegenüber Bz-Pro-Phe-Arg-NA auf kleiner 10% im Vergleich mit einem Wildtypstamm herabgesetzt ist.

### Nachweis der Carboxypeptidase Y-Defizienz (prc1)

Keine Hydrolyse durch Zellextrakte von 0,5 mmol/l Benzoyl-L-Tyrosin-4-nitroanilid, pH 7 (Agr. Biol. Chem. 35 (1971) 658-666).

0,1 ml mit Deoxycholat aktivierter (Endkonzentration 0,5%) Rohextrakt wurden mit 1 ml 0,1 mol/l Phosphatpuffer pH 7,0 und 0,2 ml 3 mmol/l Benzoyl-L-Tyrosin-4-nitroanilid (gelöst in Dimethylformamid) bei 25°C inkubiert. Nach 10 Minuten wurde die Reaktion mit 1 ml 1 mmol/l Quecksilberchlorid gestoppt und das freigesetzte p-Nitroanilin bei 410 nm bestimmt.

Eine Carboxypeptidase Y-Defizienz liegt vor, wenn. die spezifische hydrolytische Aktivität gegenüber Benzoyl-L-Tyrosin-4-nitroanilid auf kleiner 5% im Vergleich mit einem Wildtypstamm herabgesetzt ist.

### Nachweis der Carboxypeptidase S-Defizienz (cps1)

Keine Hydrolyse durch Zellextrakte von Cbz-Gly-Leu (Benzyloxycarbonyl-glycyl-L-leucin) bei pH 7,4 mit nachfolgender Analyse des freigesetzten Leucins in einem L-Aminosäure Oxidase-Peroxidase Test (Eur. J. Biochem. 73 (1977) 553-556).

0,5 ml Testlösung (0,25 mg/ml L-Aminosäure Oxidase, 0,4 mg/ml Meerrettich Peroxidase und 0,5 mmol/l MnCl₂), 0,4 ml 27,5 mmol/l Cbz-Gly-Leu Lösung (gelöst in 0,2 mol/1 Phosphatpuffer, pH 7,0), 0,05 ml o-Dianisidindihydrochlorid (2 mg/ml, gelöst in H₂O), 0,05 ml 22 mmol/l Phenylmethylsulfonylfluorid und 0,1 ml dialysiertes Zellysat (Dialyse: 0,1 M Imidazolchlorid, pH 5,3; 24 Stunden; 25°C) gemischt und die Extinktionsänderung bei 405 nm bestimmt.

Eine Carboxypeptidase Y-Defizienz liegt vor, wenn die spezifische hydrolytische Aktivität gegenüber Cbz-Gly-Leu auf kleiner 5% im Vergleich mit einem Wildtypstamm herabgesetzt ist.

Anhand der beschriebenen Nachweise wurde festgestellt, daß der Stamm ABYSDMAL81 defizient an den Proteasen A, B, D und den Carboxypeptidasen Y und S und der Stamm ABYSMAL81 defizient an den Proteasen A, B und den Carboxypeptidasen Y und S ist.

### Nachweis der Maltoseverwertungs-Auxotrophie

Kein Wachstum auf synthetischem Komplettmedium I mit 0,67% yeast nitrogen base (YNB, Salz-Vitamingemisch, Difco), 0,5% Casaminosäuren (CAA, Proteinhydrolysat, Difco), 2% Maltose (einzige C-Quelle), 20 mg/l Uracil und 30 mg/l Adenin.

### Nachweis der Uracil-Auxotrophie

Kein Wachstum auf synthetischem Komplettmedium II mit 0,67% YNB, 0,5% CAA, 2% Glucose (einzige C-Quelle) und 30 mg/l Adenin.

### Nachweis der Lysin-Auxotrophie

Kein Wachstum auf synthetischem Komplettmedium II mit Uracil (20 mg/l) aber ohne Lysin (anstelle von 0,5% CAA wurde ein Aminosäuregemisch ohne Lysin benutzt).

### Nachweis der Histidin- und Adenin-Prototrophie

Wachstum auf synthetischem Komplettmedium II mit Uracil (20 mg/l) aber ohne Adenin und ohne Histidin (anstelle von 0,5% CAA wurde ein Aminosäuregemisch ohne Histidin benutzt).

### Beispiele 2 und 3

Zur Herstellung der Saccharomycesstämme ABYSD91 (leu2-3,2-112 trp1-289a pra1 prb1 prd1 prc1 cps1), ABYSD106 (ura3-52 leu2-3,2-112 his pra1 prb1 prd1 prc1 cps1), ABYS91 (leu2-3,2-112 trp1-289a pra1 prb1 prc1 cps1) und ABYS106 (ura3-52 leu2-3,2-112 his pra1 prb1 prc1 cps1) wurde wie im Beispiel 1 beschrieben, der Saccharomyces cerevisiae-Stamm ABYSD-11 mit dem Saccharomyces carlsbergensis-Stamm DBY746, DSM 4316, gekreuzt. Nach Sporulation wurden die Hefesegreganten auf ihre Protease-Defizienzen und Auxotrophien überprüft.

### Für ABYS91 und ABYSD91:

### Nachweis der Protease-Defizienzen

### Siehe Beispiel 1

### Nachweis der Leucin und Tryptophan-Auxotrophie

Kein Wachstum auf synthetischem Komplettmedium II mit Uracil, aber ohne Leucin bzw. Tryptophan (anstelle von 0,5% CAA wurde ein Aminosäuregemisch ohne Leucin bzw. Tryptophan benutzt).

### Nachweis der Uracil-, Adenin-, Histidin- und Lysin-Prototrophie

Wachstum auf synthetischem Komplettmedium II ohne Adenin, Uracil, Histidin und Lysin (anstelle von 0,5% CAA wurde ein Aminosäuregemisch ohne Histidin und Lysin verwendet).

### Für ABYS106 und ABYSD106

### Nachweis der Protease-Defizienzen

### Siehe Beispiel 1

### Nachweis der Uracil-Auxotrophie

Kein Wachstum auf synthetischem Komplettmedium II.

### Nachweis der Leucin- und Histidin-Auxotrophie

Kein Wachstum auf synthetischem Komplettmedium II mit Uracil aber ohne Leucin bzw. Histidin (anstelle von 0,5% CAA wurde ein Aminosäuregemisch ohne Leucin bzw. Histidin verwendet).

### Nachweis der Adenin-, Lysin- und Tryptophan -Prototrophie

Wachstum auf synthetischem Komplettmedium II mit Uracil, aber ohne Adenin, Lysin und Tryptophan (anstelle von 0,5% CAA wurde ein Aminosäuregemisch ohne Lysin und Tryptophan benutzt).

### Beispiel 4

### Expression von α-Glucosidase PI

Der Saccharomyces-Stamm ABYSMAL81 (Beispiel 1) wurde mit dem Plasmid YEp/5C6b3 transformiert (Nature 275 (1978) 104-109).

Zur Herstellung dieses Plasmids wurde der Vektor YRp/GLUPI, DSM 4173P, mit den Restriktionsendonukleasen SspI und HindIII verdaut, das ca. 3,0 kBp lange SspI/-HindIII-Fragment isoliert und in das isolierte PvuII/-SpHI-Vektorfragment aus YEp 24 (Gene 8 (1979), 17-24; Cold Spring Harbor, Symp. Quant. Biol. 43 (1979) 77-90; Gene 29 (1984) 113-124; Nature 286 (1980) 860-865) - nach Auffüllung des überhängenden 5'-Endes der HindIII- und Abbau des überhängenden 3'-Endes der SphI-Restriktionsschnittstelle mit Klenow-Polymerase - ligiert. In dem entstandenen Plasmid YEp/S4 ist die α-Glucosidase PI-Expressionscassette in gleichläufiger Orientierung zum β-Lactamasegen integriert. Danach wurde in die BamHI-Restriktionsschnittstelle von YEp/S4 ein das MAL2-8^{C}p-Gen enthaltendes ca. 3,1 kBp langes BamHI-Fragment ligiert. Dazu wurde das Plasmid pRM2, DSM 4314P, mit der Restriktionsendonuklease SalI verdaut, die überhängenden 5'-Enden mit Klenow-Polymerase aufgefüllt, mit BamHI-Linkern (d(CGGGATCCCG)) versehen, mit BamHI nachgespalten und das 3,1 kBp lange MAL2-8^{C}p-Gen enthaltende BamHI-Fragment isoliert. Die entstandene Vektorkonstruktion wurde mit YEp/5C6b3 bezeichnet.

Der transformierte Stamm wurde in YEP-Medium (1 % Hefeextrakt, 2 % Pepton) mit 4 % Maltose angezogen und bis zur spät-logarithmischen bzw. stationären Phase gezüchtet. Anschließend wurde die Biomasse geerntet und mit 10 mmol/l Phosphatpuffer, pH 6,8 gewaschen. Die Zellen aus 5 ml YEP-Medium (ca. 0,1 bis 0,2 g Hefe, Naßgewicht) wurden durch Homogenisieren mit einem Whirlmix aufgeschlossen (MGG 145 (1976) 327-333).

Die Bestimmung der spezifischen α-Glucosidaseaktivität erfolgte anhand der Hydrolyse von p-Nitrophenyl-α-D-Glucopyranosid (MGG 151 (1977) 95-103) und der Proteinbestimmung nach Zamenhof (Methods Enzymol. 3 (1957) 702).

In dem auf diese Weise erhaltenen Rohextrakt war das Enzym über 10 Tage bei 4°C stabil. Auch in der SDS-Gelelektrophorese wurde über diesen Zeitraum keine Veränderung des Bandenmusters gefunden. Dies zeigt, daß auch die anderen, in diesem Überstand enthaltenen Enzyme und Proteine, in diesem Hefestamm stabil sind und nicht merklich proteolytisch abgebaut werden.

In der Tabelle I wird die enzymatische Stabilität der α-Glucosidase von Bäckerhefe (bezogen von Deutsche Hefewerke Nürnberg, DHW) mit der Stabilität von rekombinanter exprimierter α-Glucosidase in Protease-defizienten α-Glucosidase Transformanten verglichen. In Bäckerhefe erreicht die spezifische α-Glucosidase-Aktivität in der spätlogarithmischen bis frühstationären Wachstumsphase ein Maximum. Im weiteren Verlauf der Fermentation sinkt die spezifische α-Glucosidase-Aktivität merklich ab (Tabelle 1). Im Gegensatz dazu wird überraschenderweise selbst nach Erreichen der stationären Wachstumsphase die α-Glucosidase in transformierten Protease-defizienten malO-Stämmen stabil akkumuliert, wodurch die Fermentation und Aufarbeitung der Biomasse wesentlich.vereinfacht wird.

Fermentationsmedien: Bäckerhefe: 1 % Hefeextrakt, 2 % Pepton, 2 % Maltose.

ABYSMAL81: Synthetisches Komplett-Medium II.

### Beispiel 5

Heterologe Expression eines Fusionsproteins, bestehend aus dem N-terminalen Teil der α-Glucosidase und HIV1-Antigenen, in Protease-defizienten Hefestämmen.

In dem α-Glucosidase PI-Expressionsvektor YEp/5C6b3 (Beispiel 4) wurde das 1,4 KBp lange BglII-Fragment, das für ca. 80% der α-Glucosidase PI codiert, gegen ein ca. 300 Bp langes DNA-Fragment, das für einen Teil des gp41-Membranproteins des HIV1-Retrovirus codiert, ausgetauscht. Dazu wurde ein ca. 300 Bp langes RsaI/HindIII-Fragment (Sequenz vgl. Sequenz von WMJ-1 von Pos. 1638 bis Pos. 1943 aus Fig. 1 von Cell 45 (1986) 637-648) in den mit HincII und HindIII verdauten E.coli-Vektor pUC18 (M13mp18 und pUC19, Sequenz in Gene 33 (1985) 103-119) subcloniert (Konstruktion: pUC18HRH.300). Aus pUC18HRH.300 wurde das ca. 320 Bp lange BamH1/HindIII-Fragment isoliert und in das ca. 5,2 KBp lange pUR278 BamH1/HindIII-Vektorfragment ligiert (Sequenz in EMBO 2 (1983) 1791-1794) (Konstruktion: pUR278HRH.300). Das Plasmid pUR278HRH.300 wurde mit HindIII verdaut, die überhängenden 5'-Enden mit "Klenow Polymerase" aufgefüllt und mit BamHI-Linkern (d(GGGATCCC)) versehen. Danach wurde mit BamHI nachgespalten, das ca. 300 Bp lange BamHI-Fragment isoliert und in das ca. 11 KBp lange YEp/5C6b3 BglII-Vektorfragment ligiert. Bei richtiger Orientierung der gp41-Membranpolypeptid-DNA 'entsteht ein Fusionsprotein, bestehend aus dem N-Terminus der α-Glucosidase (50 Aminosäuren), 4 konstruktionsbedingten Aminosäuren an der Fusionsstelle, 101 Aminosäuren des gp41-Membranproteins und 3 konstruktionsbedingten Aminosäuren am C-Terminus mit einem Molekulargewicht von ca. 18500 D. Die gewünschte Konstruktion wurde über das exprimierte Fusionsprotein in dem Protease-defizienten Hefeexpressionsstamm ABYSMAL81 nach Transformation und Anzucht (vgl. unten) gefolgt von SDS-Gelelektrophorese und Westernblot anhand von Immunreaktivität mit humanen HIV1-Seren isoliert. Das Fusionsprotein wurde zu ca. 5% des Gesamtproteins exprimiert und war als dominante Bande in SDS-Polyacrylamidgelen nach Coomassie-Anfärbung sichtbar.

Zur Expression des α-Gluc.PI-gp41-Fusionsproteins wurden die Transformanten auf Selektivmedium (0,67% YNB, 0,5% CAA, 30 mg/l Adenin) mit 2% Glucose und 2% Maltose angezogen. Nach einer Induktionsphase von 10 bis 20 Stunden (nach Glucoseverbrauch) wurden die Zellen geerntet.

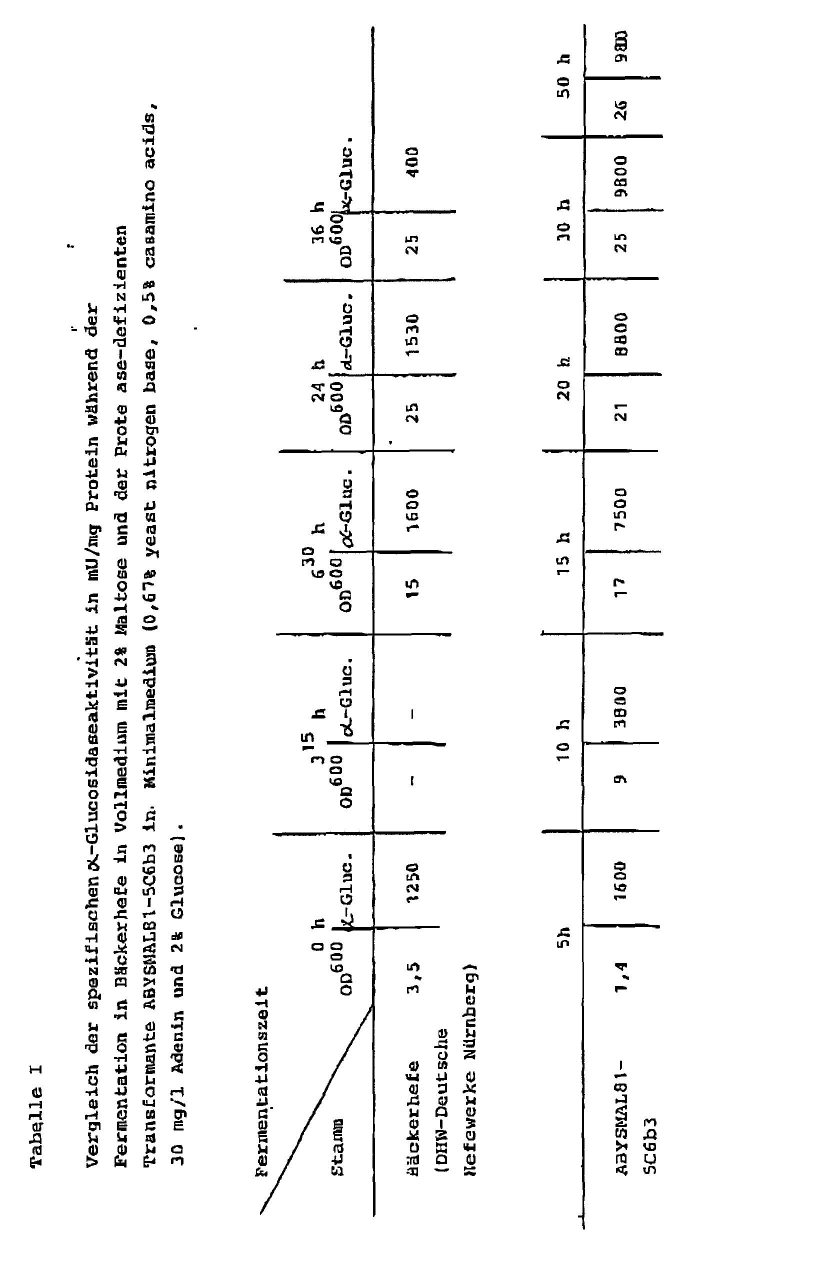

## Patentansprüche

1. Verfahren zur Herstellung von Proteinen oder proteinhaltigen Genprodukten durch Transformation von eukaryontischen Wirtszellen mit einem das Gen für das gewünschte Protein enthaltenden rekombinanten DNA-Molekül, Kultivierung der Zellen und Isolierung des Genproduktes nach der Expression, **dadurch gekennzeichnet, daß** man als Wirtszellen einen Hefestamm verwendet, der in der stationären Züchtungsphase defizient an den Proteasen A und B ist.

2. Verfahren nach Anspruch 1, **dadurch gekennzeichnet, daß** der Hefestamm zusätzlich defizient an der Protease D ist.

3. Verfahren nach Anspruch 1 oder 2, **dadurch gekennzeichnet, daß** der Hefestamm zusätzlich defizient an den Carboxypeptidasen Y oder/und S ist.

4. Verfahren nach Anspruch 3, **dadurch gekennzeichnet, daß** man den Hefestamm ABYSD-11, DSM 4322, verwendet.

5. Verfahren nach einem der Ansprüche 1 bis 4, **dadurch gekennzeichnet, daß** man den Prrotease-defizienten Hefestamm mit einem anderen, auxotrophen oder/und chemikaliensensitiven Hefestamm kreuzt, nach Sporulation und Selektionierung aus den entstandenen Hybridstämmen über die Auxotrophie oder/und Chemikaliensensitivität einen Hefestamm isoliert, der mindestens defizient an den Proteasen A und B ist und mindestens eine der Auxotrophien oder/und Chemikaliensensitivitäten der Elternstämme aufweist und diesen Hybridstamm als Wirtszellen verwendet.

6. Verfahren nach Anspruch 5, **dadurch gekennzeichnet, daß** man den Hefestamm ABYSD-11, DSM 4322, mit dem Stamm TCY2824-1A, DSM 4317, oder DBY746, DSM 4316, kreuzt, einen Stamm. der defizient an den Proteasen A, B und den Carboxypeptidasen Y und S und gegebenenfalls der Protease D ist, sowie die Uracil-, Lysin- und Maltoseverwertungsauxotrophien, die Leucin- und Tryptophan- oder die Leucin-, Uracil- und Histidin-Auxotrophien der Elternstämme aufweist, isoliert und als Wirtszellen verwendet.

7. Verfahren nach einem der Ansprüche 5 oder 6, **dadurch gekennzeichnet, daß** man ein rekombinantes DNA-Molekül verwendet, welches zusätzlich ein oder mehrere Gene enthält, die die Auxotrophien oder/und Chemikaliensensitivitäten des Wirtsstammes komplementieren.

8. Verfahren nach einem der Ansprüche 1 bis 7, **dadurch gekennzeichnet, daß** man als rekombinantes DNA-Molekül ein Plasmid, einen Integrationsvektor oder ein integrierendes DNA-Fragment verwendet.

9. Verfahren nach Anspruch 8, **dadurch gekennzeichnet, daß** das das rekombinante DNA-Molekül ein in hoher Kopienzahl in der Zelle vorkommendes Hefeplasmid ist.

10. Hefestamm, der in.der stationären Züchtungsphase defizient an den Proteasen A und B ist.

## Claims

1. Method for producing proteins or gene products containing protein by transforming eukaryotic host cells with a recombinant DNA molecule containing the gene for the desired protein, culturing the cells and isolating the gene product after expression, **characterized in that** a yeast strain which is deficient in proteases A and B in the stationary growth phase is used as the host cells.

2. Method as claimed in claim 1, **characterized in that** the yeast strain is additionally deficient in protease D.

3. Method as claimed in claim 1 or 2, **characterized in that** the yeast strain is additionally deficient in carboxypeptidases Y or/and S.

4. Method as claimed in claim 3, **characterized in that** the yeast strain ABYSD-11, DSM 4322 is used.

5. Method as claimed in one of the claims 1 to 4, **characterized in that** the protease-deficient yeast strain is crossed with another auxotrophic or/and chemical-sensitive yeast strain, after sporulation and selection of the resulting hybrid strains by means of the auxotrophy or/and chemical sensitivity, a yeast strain is isolated which is at least deficient in proteases A and B and has at least one of the auxotrophies or/and chemical sensitivities of the parent strains and this hybrid strain is used as the host cells.

6. Method as claimed in claim 5, **characterized in that** the yeast strain ABYSD-11, DSM 4322 is crossed with the strain TCY2824-1A, DSM 4317 or DBY746, DSM 4316, a strain which is deficient in proteases A, B and the carboxypeptidases Y and S and optionally protease D and has the uracil, lysine and maltose utilization auxotrophies, the leucine and tryptophan or leucine, uracil and histidine auxotrophies of the parent strains is isolated and used as host cells.

7. Method as claimed in one of the claims 5 or 6, **characterized in that** a recombinant DNA molecule is used which additionally contains one or more genes which complement the auxotrophies or/and chemical sensitivities of the host strain.

8. Method as claimed in one of the claims 1 to 7, **characterized in that** a plasmid, an integration vector or an integrating DNA fragment is used as the recombinant DNA molecule.

9. Method as claimed in claim 8, **characterized in that** the recombinant DNA molecule is a yeast plasmid which occurs in a high copy number in the cell.

10. Yeast strain which is deficient in proteases A and B in the stationary growth phase.

## Revendications

1. Procédé pour la préparation de protéines ou de produits de gène contenant des protéines par transformation de cellules-hôtes eucaryontiques avec une molécule d'ADN recombinant contenant le gène pour la protéine souhaitée, culture des cellules et isolement du produit de gène après l'expression, **caractérisé en ce qu'**on utilise, comme cellules-hôtes, une souche de levure qui, dans la phase de culture stationnaire, est déficiente au niveau des protéases A et B.

2. Procédé selon la revendication 1, **caractérisé en ce que** la souche de levure est en outre déficiente en la protéase D.

3. Procédé selon la revendication 1 ou 2, **caractérisé en ce que** la souche de levure est en outre déficiente au niveau des carboxypeptidases Y ou/et S.

4. Procédé selon la revendication 3, **caractérisé en ce qu'**on utilise la souche de levure ABYSD-11, DMS 4322.

5. Procédé selon l'une quelconque des revendications 1 à 4, **caractérisé en ce qu'**on croise la souche de levure déficiente en protéases avec une autre souche de levure auxotrophe ou/et sensible aux agents chimiques, on isole, après sporulation et sélection à partir des souches hybrides formées par l'intermédiaire de l'auxotrophie ou/et de la sensibilité aux agents chimiques, une souche de levure qui est au moins déficiente au niveau des protéases A et B et présente au moins une des auxotrophies ou/et sensibilités aux agents chimiques des souches parentes et on utilise cette souche hybride comme cellules-hôtes.

6. Procédé selon la revendication 5, **caractérisé en ce qu'**on croise la souche de levure ABYSD-11, DMS 4322, avec la souche TCY2824-1A, DMS 4317, ou DBY746, DMS 4316, on isole une souche qui est déficiente au niveau des protéases A, B et au niveau des carboxypeptidases Y et S, et le cas échéant, la protéase D, et présente les auxotrophies de l'uracil, de la lysine et du maltose, les auxotrophies de la leucine et du tryptophane ou les auxotrophies de la leucine, de l'uracil et de l'histidine des souches parentes, et on les utilise comme cellules-hôtes.

7. Procédé selon l'une ou l'autre des revendications 5 ou 6, **caractérisé en ce qu'**on utilise une molécule d'ADN recombinant qui contient, en outre, un ou plusieurs gènes qui complètent les auxotrophies ou/et les sensibilités aux agents chimiques de la souche-hôte.

8. Procédé selon l'une quelconque des revendications 1 à 7, **caractérisé en ce qu'**on utilise comme molécule d'ADN recombinant un plasmide, un vecteur d'intégration ou un fragment d'ADN intégrant.

9. Procédé selon la revendication 8, **caractérisé en ce que** la molécule d'ADN recombinant est un plasmide de levure se trouvant dans la cellule en un nombre de copies élevé.

10. Cellule de levure qui est déficiente dans la phase de culture stationnaire au niveau des protéases A et B.
